# EUROPEAN PATENT SPECIFICATION

(11) **EP 4 554 641 B1**
(45) Date of publication and mention of the grant of the patent: **03.06.2026**
(21) Application number: 23761568.7
(22) Date of filing: 14.07.2023
(51) Int. Cl.: A61M 5/32

(54) **SAFETY SHEATH AND NEEDLE ASSEMBLY WITH SAFETY SHEATH**
SICHERHEITSHÜLSE UND NADELANORDNUNG MIT SICHERHEITSHÜLSE
GAINE DE SÉCURITÉ ET ENSEMBLE AIGUILLE DOTÉ D'UNE GAINE DE SÉCURITÉ

(30) Priority: 15.07.2022 PL 44174322
(43) Date of publication of application: 21.05.2025
(73) Proprietor: Niesiobedzki, Krzysztof, 22885 Barsbuttel (DE); Niesiobedzki, Michael, 22885 Barsbuttel (DE)
(72) Inventor: NIESIOBEDZKI, Krzysztof, 22885 Barsbuettel (DE); NIESIOBEDZKI, Michael, 22885 Barsbuettel (DE)
(74) Representative: Ptasinski, Konrad Witold
(86) International application number: PCT/IB2023/057216
(87) International publication number: WO 2024/013708

(56) References cited:
- CN-A- 107 737 391
- US-A1- 2015 273 159
- US-A1- 2020 046 276

## Description

The object of the invention is a safety sheath and a needle assembly with a safety sheath for use in the practice of several types of injections, such as an intramuscular, intravenous, subcutaneous injection. The objects of the invention are used to ensure safe transportation and distribution of injection needles.

The injection is used when treating patients by introducing a drug solution into the tissue using a needle connected to a syringe or another medical device. It can also be used to diagnose many medical conditions through collecting patient material for examination by puncturing a vein and taking a blood sample. Given the fact that many diseases are contracted indirectly through contact with contaminated objects or the patient's blood, the aspect of ensuring the safety of medical staff when using needles is particularly important. After injection, the needle becomes medical waste and must be transported to its designated disposal site. When handling a used needle, there is a risk of accidentally puncturing medical personnel and thus exposing them to contracting dangerous diseases. Furthermore, there is also the possibility of accidental puncture by medical staff before the needle is used. In such a case, the needle cannot be used because it is no longer sterile and becomes waste. Medical staff must therefore be particularly careful when transporting a needle prepared for injection, which significantly slows down and hinders their work.

The prior art needle safety caps protect medical personnel from accidental puncture while transporting a used needle. Solutions disclosed in the prior art often have a complex design and are unintuitive to use. Moreover, once the needle has been put into the safe position, it is difficult, and often impossible, to put the needle back into the open position.

The European Patent EP0832660B1 discloses a needle set comprising a needle and a cap with an opening wherein the needle is placed, the cap being mounted on a container holding a fluid. The set has a longitudinal sheath comprising the proximal and distal end and side walls with an inner surface that defines a longitudinal cavity. The set is provided with a hinge with a fixing element for the swinging connection of the sheath to the cap. The sheath may thus oscillate between an initial closed position, an open position, and a locked position. Additionally, the set includes a removable longitudinal cover that couples with the sheath to maintain the initial closed position. The latched position, on the other hand, is obtained by the presence of a latch such that the cover is latched in the fixing element.

US5490841A relates to a safe protection device for needles. The disclosed device comprises an elongated housing with a longitudinal needle entrance that is defined between the side walls of the housing. At least one hinge member extending transversely with respect to the longitudinal entrance for closing the needle inside the housing is provided. In one embodiment the housing is additionally provided with an element in the form of a longitudinal flap attached flexibly to one sidewall such that after the needle is inserted into the housing, it is locked in its inner region.

A needle sheath assembly with safety aids is known from the specification of the European patent document EP0724890B1. The set includes a needle cannula that has a proximal end and a distal end. A cap is also provided that is connected to the medical device by its proximal end, while its distal end is connected to the proximal end of the needle cannula. A base element is attached to the cap, which is hingedly connected to the proximal end of the needle sheath. The needle sheath includes an elongated opening and may rotate from the first position wherein at least the distal end of the needle cannula is exposed to the second position wherein the needle cannula is inside the elongated aperture of the needle sheath. Furthermore, a sleeve that has a longitudinal slot is pivotally connected to the needle sheath. The sleeve may rotate about the needle sheath such that its longitudinal slot is aligned with the longitudinal opening of the needle sheath to obtain an open position. When the longitudinal slot of the sleeve does not coincide with the longitudinal opening of the needle sheath, a locked position is obtained.

The European patent document EP3976141A1 discloses a needle housing and a safety set including a needle housing. The needle housing comprises a mounting portion for fixing the needle and a cover portion hingedly connected to the mounting portion. The cover portion is movable and may be arranged in the open position, i.e., the first housing position and the second housing position. In the first housing position, the cover portion is detachably connected to the mounting portion, while in the second housing position, the cover portion is irreversibly connected to the mounting portion. To provide the first and second positions of the housing, the cover portion is provided with appropriate coupling elements.

The document US2020/046276A1 discloses an arterial syringe assembly comprising a housing with a proximal end configured to couple with a syringe barrel and a distal end configured to couple with a needle. A needle cover is hinged to the housing and can be positioned axially relative to the needle, with a needle plug at its distal end allowing the needle tip to extend into the plug material. A hydrophobic filter is arranged within the syringe barrel to evacuate air through its proximal end.

The document US2015/273159 A1 discloses a safety injection needle with a safety sheath attached to the needle base. The sheath rotates about an elastic telescopic plate and includes safety hooks cooperating with hooks on the needle base. The safety mechanism is integral with the needle base and provides enhanced protection compared to conventional devices.

The document CN107737391A discloses a safety injection needle with a protective cover. The needle comprises a needle body with a stepped needle base and a needle tube, while the protective cover includes a U-shaped grooved tube with a needle locking structure and a hinged connection to a fixing portion attached to the needle base. The cover prevents medical staff from accidental punctures and is firmly and reliably secured to the needle base.

The technical problem faced by the present invention is to propose such a safety sheath and a needle assembly with a safety sheath that will ensure safe transportation and distribution of the needle without exposing medical personnel to a puncture. The ability to temporarily secure the needle with the possibility of returning it to the open position is also desired. Moreover, it is desirable that the handling of the safety sheath can be carried out with one hand. In addition, it is desirable that the secured needle is positioned stationary and stable without the risk of accidentally returning to the open position. Moreover, it is desirable that the safety sheath is easy to manufacture and has a simple structure to ensure its reliability of operation. It is also desirable that the safety sheath can be used for other suitable medical devices already available on the market.

The first object of the invention is a safety sheath according to the appended claim 1.

Preferably, the longitudinal lock is arranged on the inner surface of two opposing side walls of the body.

Preferably, the longitudinal lock extends along the entire length of the inner surface of the side wall of the body.

Preferably, the body has a cross section in the shape of a rectangle or a trapezoid.

Preferably, the needle fixing element comprises an assembly sleeve attached to the fixing element at its first end.

Preferably, longitudinal splines are disposed on the inner surface of the sleeve.

Preferably, a cap is inserted into the other end of the sleeve.

Preferably, the front wall of the body is truncated towards the upper wall of the body.

Preferably, the hinge comprises a flexible element.

Preferably, a projection is arranged on the upper wall of the body.

Preferably, elongated extrusions are arranged on the outer side surfaces of the body walls and/or on the front wall of the body and/or on the projection.

Preferably, the safety sheath is an integral plastic component.

The second object of the invention is a needle assembly with a safety sheath for fixing to the syringe connector according to the appended claim 13.

Preferably, the sleeve and cap are an integral element or are separate elements fixed in one another.

Preferably, the cap is an injection needle connector.

The safety sheath and the needle and safety sheath assembly ensure that the needle can be safely and conveniently transported and distributed. The safety sheath includes a body with a longitudinal slot designed for inserting the needle, wherein at least one safety catch is arranged inside the body, which allows the needle to be locked and kept permanently in a safe position. Additionally, at least one elongated lock is disposed on the inner surface of at least one side wall of the body. The elongated lock is located in a plane between the longitudinal slot and the safety catch and provides the possibility of placing the needle in an intermediate position, the so-called transport position, between the safe position and the open position, wherein the needle is introduced into the internal space of the body of the safety sheath and its sharp end does not protrude beyond the safety sheath. Moreover, because of the rounded end of the longitudinal edges of the elongated lock, it is possible to manipulate the needle from the open position to the transport position and vice versa. In a preferred embodiment of the invention, the elongated lock extends on the inner surface of both side walls along its entire length, so that the needle placed in the transport position is protected against accidental ejection and injury to the user. In addition, the presence of two opposite locks facilitates the insertion of the needle into the transport position so that the hinge is not subjected to lateral deformations and thus the reliability of the structure is increased.

Individual needle positions are obtained by manipulating the body of the safety sheath relative to the fixing element. This movement is a swinging movement and is possible due to the presence of a hinge that connects the body with the fixing element. In addition, the hinge is equipped with a flexible element, so that changing the needle position between individual positions is facilitated by this flexible element, resulting in smooth movement and user comfort. Such a safety sheath structure ensures that the needle can be freely repositioned between the open position and the transport position and vice versa, and that the needle can ultimately and irreversibly be placed in the safety position.

In a preferred embodiment of the invention, the cross section of the body of the safety sheath has a trapezoidal shape, which allows the needle to be operated with one hand.

The fixing element comprises an assembly sleeve which is attached to the fixing element at its first end. In addition, longitudinal splines are disposed on the inner surface of the sleeve. The presence and structure of the sleeve ensures that the safety sheath can be permanently connected to the modular connector so that it does not rotate with respect to the sleeve.

The front wall of the body is truncated towards the upper wall of the body, while a projection is placed on the upper wall of the body. What is important is that elongated extrusions are arranged on the outer side surfaces of the body walls and/or on the front wall of the body and/or on the projection. This structure allows the safety sheath to be easily operated by the user with one hand, which has a significant effect on the comfort and safety of use.

The safety sheath is a homogeneous component that is made of plastic in the injection moulding process. The manufacture of the safety sheath is therefore fast, simple, and economically advantageous.

The needle set with the safety sheath offers the possibility of placing the needle in three positions: open, transport and safe. In the open position, the hinge is open such that the wall of the fixing element with the opening is arranged substantially perpendicular to the body of the safety sheath and the needle is arranged substantially parallel to the body of the safety sheath. The transport position is an intermediate position and is reached when the needle engages with the elongated lock but does not pass further through the catch. As a result, the needle rests against the surface of the elongated lock, and changing the position back to the open position requires the use of a specific force that is intended to deform the safety sheath (side wall and/or hinge) and allow the needle to pass through the slot in the body. In the safe position, the needle is hooked so that its position cannot change and return to the transport position or the open position.

The basic functionality of the safety sheath is the possibility of introducing it into an intermediate position, the so-called transport position. The transport position gives the possibility of temporarily securing the needle, and then placing it again in the open position. If necessary, it is therefore possible to move the needle from the open position to the transport position and back again for an essentially unlimited number of times. The shape of the body and the presence of extrusions ensure that the safety sheath can be operated with one hand, which significantly contributes to the comfort and efficiency of the medical staff.

The solution of the present invention is set forth in the following embodiments and illustrated in the drawings, wherein Fig. 1a shows a side view of the safety sheath according to the first embodiment, Fig. 1b shows a bottom view of the safety sheath of Fig. 1a, Fig. 2a shows a side view of the safety sheath according to the second embodiment, Fig. 2b shows a bottom view of the safety sheath of Fig. 2a, Fig. 2c shows a cross section of the safety sheath of Fig. 2a and 2b, Fig. 3a shows a side view of the safety sheath according to the third embodiment, Fig. 3b shows a bottom view of the safety sheath of Fig. 3a, Fig. 3c shows a plan view of the safety sheath of Fig. 3a and 3b, Fig. 3d shows a front (face) view of the safety sheath of Fig. 3a-3c, Fig. 3e shows a cross section of the body of the safety sheath of Fig. 3a, Fig. 3f shows a cross-section of the body of the safety sheath according to the alternative embodiment of the invention, Fig. 4 shows a bottom view of the safety sheath according to the third embodiment with the lock extending over the entire length of the body, Fig. 5 shows an axonometric view of the safety sheath according to the third embodiment (without a cap), Fig. 6a shows the needle assembly with the safety sheath in the open position, Fig. 6b shows the needle assembly with the safety sheath in the transport position, Fig. 6c shows the needle assembly with the safety sheath in the safe position.

### Example 1

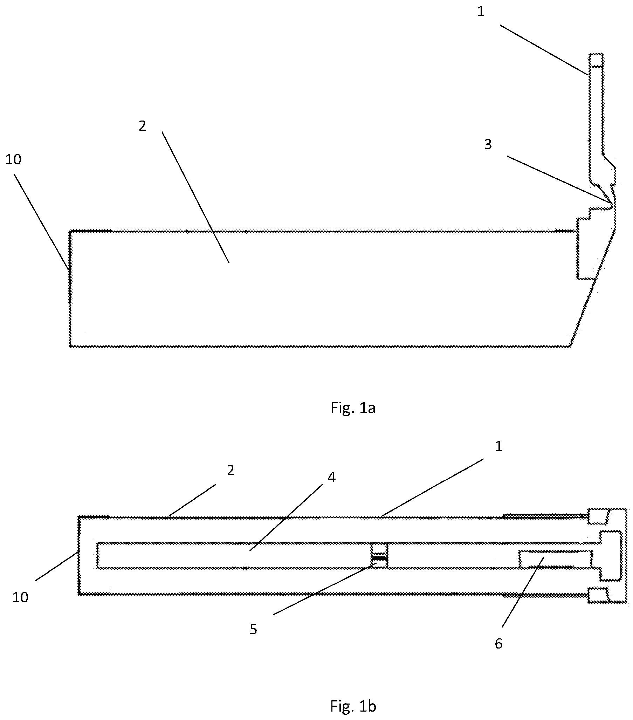

The first embodiment of the safety sheath of the present invention is illustrated in schematic views in Fig. 1a-1b. As illustrated in the figures, the safety sheath includes a needle fixing element 1 and a body 2 that is attached to the fixing element 1 via the hinge 3. In this embodiment, the body 2 has a cuboid shape. On the lower wall of the body 2, a longitudinal slot 4 extends to receive the needle. The length of the longitudinal slot 4 must be not less than the length of the needle inserted into the safety sheath of the present invention. A single catch 5 is arranged inside the body, which is designed to ensure safe and irreversible fixing of the needle. The catch 5 comprises two locking elements that take the form of flexible tongues and are positioned on both surfaces of the inner side walls at an angle towards the upper wall of the body, with the ends of the flexible tongues overlapping. Owing to this catch 5 structure, the needle can be locked in the catch 5 to obtain a safe position by deforming the flexible tongues and passing through the catch 5 towards the upper wall of the body, whereby it is not possible change the to position of the needle to the open or transport position due to the overlapping ends of the flexible tongues and their angular orientation.

An elongated lock 6 is arranged between the catch 5 and the longitudinal slot 4 on a part of the inner surface length of one side wall of the body 2. In this embodiment, the elongated lock 6 extends from the region of the body 2 where it engages the fixing element 1 towards the front wall 10 of the body 2 for a length of about a quarter of the length of the body 2. The elongated lock 6 has a substantially rectangular shape, with its longitudinal edges facing the interior of the body 2 being rounded to facilitate the insertion of the needle into the body 2.

In the present embodiment, the elongated lock 6 extends in the direction of the centre of symmetry of the body 2 from only one wall of the body 2, wherein the elongated lock 6 extends in a lateral direction at least to an area defined by the centre of symmetry of the body 2, slightly beyond the centre of symmetry of the body 2 or a distance ranging from the side wall of the body 2 to the centre of symmetry of the body 2 reduced by the radius of the inserted needle. The needle is engaged by the lock 6 under the pressure applied to the body 2, thereby reaching the transport position. Then, the needle is placed back in the open position, under the pressure applied to the body 2 in the opposite direction.

The safety sheath is made of plastic as a single element to facilitate the manufacturing process. In the alternative embodiments of the invention, the safety sheath may be made up of several components, these components being joined together by methods known in the art.

### Example 2

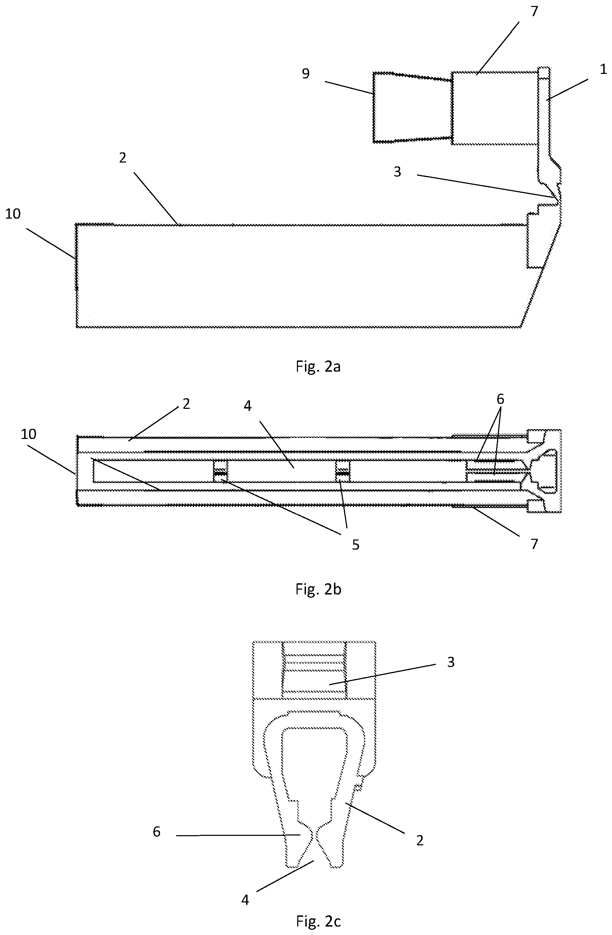

The second embodiment of the safety sheath according to the invention is shown in Fig. 2a-c. In general, the structure of the safety sheath is substantially the same as the structure of the safety sheath shown in Example 1, therefore, the corresponding design elements will not be repeated for the sake of clarity of this disclosure.

In the present embodiment, the safety sheath has the shape of a narrowed cuboid on the side of the slot 4, so that the cross-section of the body 2 has the shape similar to a trapezoid, as shown in Figs. 2c. In this embodiment, the safety sheath is provided with two elongated locks 6 arranged on the inner surfaces of two opposing side walls of the body 2, respectively. The location and length of the elongated locks 6 are substantially the same as in Example 1. The elongated locks 6, on the other hand, extend in the direction of the axis of symmetry of the body 2 at a distance smaller than the distance between the inner surface of the side wall of the body 2 and the centre of symmetry of the body 2, so that the longitudinal opening receiving the needle between the individual elongated locks 6 is maintained. Importantly, the width of the needle receiving the opening is smaller than the needle diameter. When inserting the needle through the receiving opening of the lock 6, the side walls of the body 2 deform substantially symmetrically outwards, whereby the width of the receiving opening 6 of the lock increases to accommodate the diameter of the inserted needle, allowing it to pass through the lock 6 area. Thus, the deformation of the hinge 3 is significantly reduced or eliminated, providing a more durable structure of the safety sheath.

Additionally, in this embodiment, within the body 2 there are two catches 5 arranged such that the gaps between them, between the first catch 5 and the front wall 10 of the body 2 and between the second catch 5 and the end of the elongated locks 6, are equal. The use of two catches 5 arranged at a distance along the longitudinal direction of the safety sheath allows for a more secure and stable locking of the needle in the safe position and influences the versatility of the structure due to the possibility of using needles of different lengths (from the length between the proximal end of the body 2 and the first catch 5 to the length between the proximal end of the body 2 and the front wall of the body 2).

Importantly, in this embodiment, the fixing element 1 further comprises a sleeve 7 wherein a cap 9 is fixed together with a needle for stable attachment of the safety sheath.

### Example 3

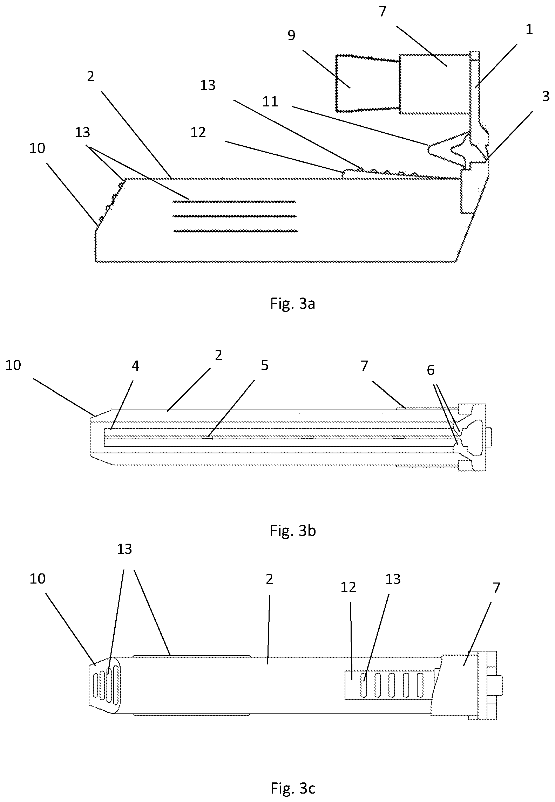

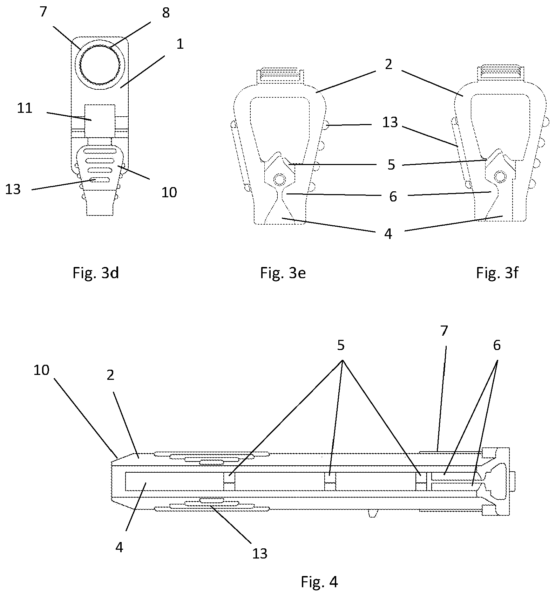

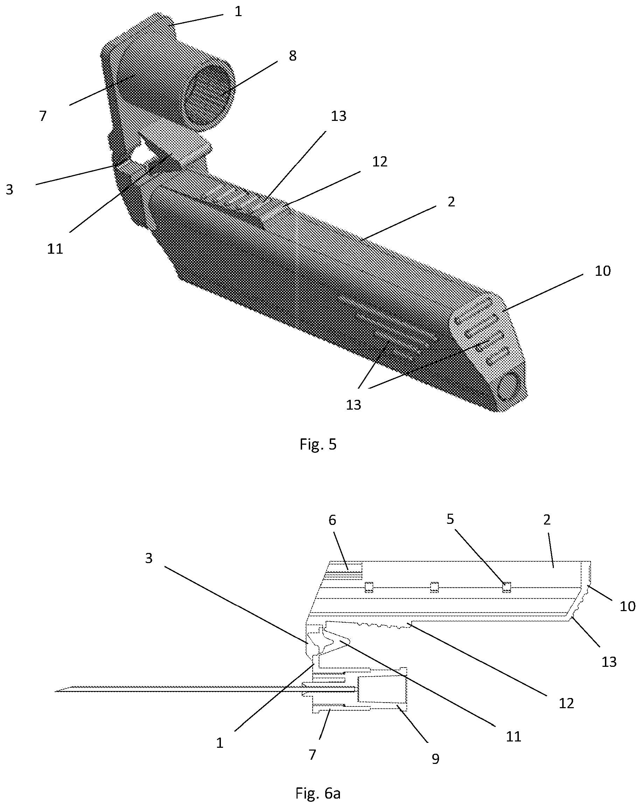

The third embodiment of the safety sheath according to the invention is shown in Fig. 3a-e and in Fig. 4 and Fig. 5, wherein in Fig. 5, the cap 9 was omitted to illustrate the internal structure of the sleeve 7, i.e., the splines 8. In addition, in Fig. 3c, the cap 9 and partially the sleeve 7 were also omitted to present a projection 12 with elongated extrusions 13. In general, the structure of the safety sheath is substantially coincident with the structure of the safety sheath shown in Example 2, therefore, the coinciding design elements will not be repeated for the sake of clarity of this disclosure.

In the present embodiment, the two elongated locks 6 extend along the entire length of the body 2, thereby obtaining a more secure and stable locking of the needle in the transport position. In the alternative embodiments of the invention, the lock 6 may extend to a shorter length, for example one quarter, as shown in Fig. 4, provided that temporary and reversible locking of the needle in the transport position is ensured. The shape of the lock 6 is shown in a cross-section in Fig. 3e, along with the indication of the needle location in the transport position of the safety sheath. In addition, Fig. 3f shows a cross-section of an alternative embodiment of the safety sheath, analogous to Fig. 3e, but with the lock 6 extending from only one side of the body 2, as implemented in Example 1.

Preferably, the front wall 10 of the body 2 is truncated towards the upper wall of the body 2. This shape of the front wall 10 makes it easy for the user to lock the needle in the safe position by pressing at an appropriate angle on the solid surface with one hand. In addition, elongated extrusions 13 are arranged on the outer side surfaces of the body 2 side walls and on the outer surface of the front wall 10 to facilitate the handling of the safety sheath with one hand. Moreover, the hinge 3 includes a flexible element 11 which also facilitates and increases the convenience of using the safety sheath with one hand by assisting the swinging movement of the hinge 3. In addition, a projection 12 is arranged on the upper wall of the body 2 which also includes elongated extrusions 13 to facilitate handling of the safety sheath with one hand.

In the solution according to the third embodiment, three catches 5 are arranged at equal intervals within the body 2, which further increases the stability and reliability of locking the needle in the safe position and influences the versatility of the structure by allowing the use of different length needles.

On the inner surface of the sleeve 7, longitudinal splines 8 are arranged to form a socket for receiving the cap 9 so that it is fixed to the sleeve 7 firmly and does not rotate therein.

### Example 4

An embodiment of a needle assembly with a safety sheath is illustrated in Fig. 6a-6c. The needle assembly with the safety sheath includes an injection needle with a cap 9 and a safety sheath. The injection needle cap 9 is a connecting element for the injection needle itself. In this embodiment, the safety sheath is the sheath according to the third embodiment, however, in the alternative embodiments of the invention, the needle assembly is adapted to cooperate with the safety sheath of any embodiment of the present disclosure. In addition, in this embodiment, the sleeve 7 and the cap 9 are separate components designed for detachable abutment fixing to provide a stable and stationary fixing of the injection needle to the safety sheath. However, in alternative embodiments, they may be an integral component produced in a single manufacturing process.

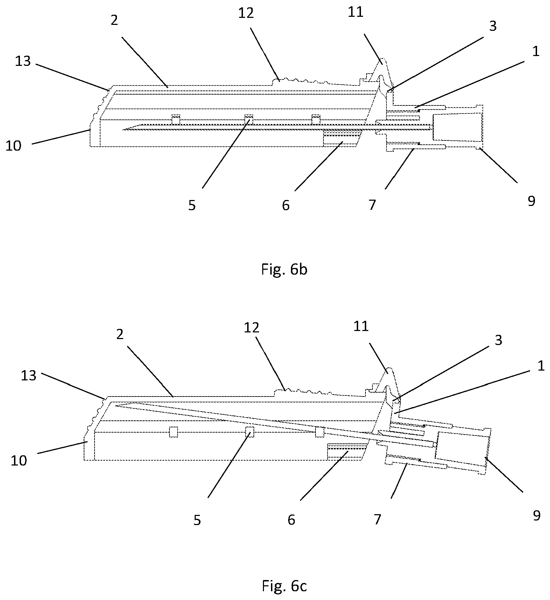

As illustrated in Fig. 6a, the needle may be arranged in the open position so that it is arranged parallel to the body 2 of the safety sheath. Then, using one hand, the needle is placed in the transport position (Fig. 6b), wherein a portion of the injection needle pin is positioned in the area between the lock 6 and the catch 5, thus avoiding the accidental exposure of the sharp end of the needle. It is possible to move the needle from the transport position back to the open position and vice versa. When more pressure is applied to the safety sheath, the needle is moved into the safe position so that it is locked by at least one catch 5, whereby it is not possible to unlock it and bring it back into the transport or open position. The Fig. 6c. shows the needle in the safe position.

### List of references:

1 - fixing element,
2 - body,
3 - hinge,
4 -slot,
5 - catch,
6 - lock,
7 - sleeve,
8 - splines,
9 - cap,
10 - front wall,
11 - flexible element,
12 - projection,
13 - extrusions.

## Claims

1. A safety sheath comprising a fixing element (1) and a body (2) connected with the fixing element (1) by a hinge (3), wherein a longitudinal slot (4) extends on the lower wall of the body (2), wherein the safety sheath includes a safe position and an open position, wherein at least one catch (5) is arranged inside the body (2) and is configured to permanently lock a needle in the safe position, **characterized in that** the safety sheath furthermore includes an intermediate position, wherein a longitudinal lock (6) is arranged on at least part of the length of the inner surface of at least one side wall of the body (2), in the plane between the catch (5) and the longitudinal slot (4) and is configured to reversibly engage the needle in the intermediate position, between the safe position and the open position.

2. The safety sheath according to claim 1, **characterized in that** the longitudinal lock (6) is arranged on the inner surface of two opposing side walls of the body (2).

3. The safety sheath according to any one of claims 1-2, **characterized in that** the longitudinal lock (6) extends along the entire length of the inner surface of the side wall of the body (2).

4. The safety sheath according to any one of claims 1-3, **characterized in that** the body (2) has a cross section in the shape of a rectangle or a trapezoid.

5. The safety sheath according to any one of claims 1-4, **characterized in that** the needle fixing element (1) includes an assembly sleeve (7) attached to the fixing element (1) at its first end.

6. The safety sheath according to claim 5, **characterized in that** longitudinal splines (8) are disposed on the inner surface of the sleeve (7).

7. The safety sheath according to any one of claims 5-6, **characterized in that** a cap (9) is inserted into the other end of the sleeve (7).

8. The safety sheath according to any one of claims 1-7, **characterized in that** the front wall (10) of the body (2) is truncated towards the upper wall of the body (2).

9. The safety sheath according to any one of claims 1-8, **characterized in that** the hinge (3) includes a resilient element (11).

10. The safety sheath according to any one of claims 1-9, **characterized in that** a projection (12) is arranged on the upper wall of the body (2).

11. The safety sheath according to claim 10, **characterized in that** elongated extrusions (13) are arranged on the outer side surfaces of the body (2) walls and/or on the front wall (10) of the body (2) and/or on the projection (12).

12. The safety sheath according to claim 10, **characterized in that** it is an integral plastic component.

13. A needle assembly with a safety sheath for fixing to the syringe connector, comprising an injection needle comprising a cap (9) and a sleeve (7) and a safety sheath **characterized in that** the safety sheath is a safety sheath according to any one of claims 1-12.

14. The needle assembly with the safety sheath according to claim 13, **characterized in that** the sleeve (7) and the cap (9) are an integral component or are separate elements fixed in one another.

15. The needle assembly with the safety sheath according to any one of claims 13-14, **characterized in that** the cap (9) is an injection needle connector.

## Patentansprüche

1. Die Sicherheitshülle, umfassend ein Befestigungselement (1) und einen Körper (2), der mit dem Befestigungselement (1) durch ein Scharnier (3) verbunden ist, wobei sich ein Längsschlitz (4) auf der unteren Wand des Körpers (2) erstreckt, wobei die Sicherheitshülle eine sichere Position und eine offene Position beinhaltet, wobei mindestens eine Sperre (5) innerhalb des Körpers (2) angeordnet ist und dazu konfiguriert ist, eine Nadel in der sicheren Position dauerhaft zu sperren, **dadurch gekennzeichnet, dass** die Sicherheitshülle ferner eine Zwischenposition beinhaltet, wobei eine Längsverriegelung (6) auf mindestens einem Teil der Länge der Innenfläche mindestens einer Seitenwand des Körpers (2) in der Ebene zwischen der Sperre (5) und dem Längsschlitz (4) angeordnet ist und dazu konfiguriert ist, die Nadel in der Zwischenposition zwischen der sicheren Position und der offenen Position reversibel in Eingriff zu nehmen.

2. Die Sicherheitshülle nach Anspruch 1, **dadurch gekennzeichnet, dass** die Längsverriegelung (6) an der Innenfläche von zwei gegenüberliegenden Seitenwänden des Körpers (2) angeordnet ist.

3. Die Sicherheitshülle nach einem der Ansprüche 1-2, **dadurch gekennzeichnet, dass** sich die Längsverriegelung (6) über die gesamte Länge der Innenfläche der Seitenwand des Körpers (2) erstreckt.

4. Die Sicherheitshülle nach einem der Ansprüche 1-3, **dadurch gekennzeichnet, dass** der Körper (2) einen Querschnitt in Form eines Rechtecks oder eines Trapezes aufweist.

5. Die Sicherheitshülle nach einem der Ansprüche 1-4, **dadurch gekennzeichnet, dass** das Nadelbefestigungselement (1) eine Montagehülse (7) beinhaltet, die an dem Befestigungselement (1) an seinem ersten Ende befestigt ist.

6. Die Sicherheitshülle nach Anspruch 5, **dadurch gekennzeichnet, dass** Längsverzahnungen (8) an der Innenfläche der Hülse (7) angeordnet sind.

7. Die Sicherheitshülle nach einem der Ansprüche 5-6, **dadurch gekennzeichnet, dass** eine Kappe (9) in das andere Ende der Hülse (7) eingesetzt ist.

8. Die Sicherheitshülle nach einem der Ansprüche 1-7, **dadurch gekennzeichnet, dass** die Vorderwand (10) des Körpers (2) zur oberen Wand des Körpers (2) hin abgestumpft ist.

9. Die Sicherheitshülle nach einem der Ansprüche 1-8, **dadurch gekennzeichnet, dass** das Scharnier (3) ein elastisches Element (11) beinhaltet.

10. Die Sicherheitshülle nach einem der Ansprüche 1-9, **dadurch gekennzeichnet, dass** ein Vorsprung (12) an der oberen Wand des Körpers (2) angeordnet ist.

11. Die Sicherheitshülle nach Anspruch 10, **dadurch gekennzeichnet, dass** an den äußeren Seitenflächen der Wände des Körpers (2) und/oder an der Vorderwand (10) des Körpers (2) und/oder an dem Vorsprung (12) längliche Vorsprünge (13) angeordnet sind.

12. Die Sicherheitshülle nach Anspruch 10, **dadurch gekennzeichnet, dass** sie ein integrales Kunststoffbauteil ist.

13. Die Nadelbaugruppe mit einer Sicherheitshülle zur Befestigung an einem Spritzenanschluss, umfassend eine Injektionsnadel mit einer Kappe (9) und einer Hülse (7) und eine Sicherheitshülle, **dadurch gekennzeichnet, dass** die Sicherheitshülle eine Sicherheitshülle nach einem der Ansprüche 1-12 ist.

14. Die Nadelbaugruppe mit der Sicherheitshülle nach Anspruch 13, **dadurch gekennzeichnet, dass** die Hülse (7) und die Kappe (9) ein integraler Bestandteil sind oder separate Elemente sind, die ineinander befestigt sind.

15. Die Nadelbaugruppe mit der Sicherheitshülle nach einem der Ansprüche 13-14, **dadurch gekennzeichnet, dass** die Kappe (9) ein Injektionsnadelanschluss ist.

## Revendications

1. Gaine de sécurité comprenant un élément de fixation (1) et un corps (2) lié au élément de fixation (1) par une charnière (3), dans laquelle une fente longitudinale (4) s'étend sur la paroi inférieure du corps (2), dans laquelle la gaine de sécurité inclut une position de sécurité et une position ouverte, dans laquelle au moins un cliquet (5) est disposé dans le corps (2) et est configuré pour verrouiller de manière permanente une aiguille dans la position de sécurité, **caractérisée en ce que** la gaine de sécurité en outre inclut une position intermédiaire, dans laquelle un verrou longitudinal (6) est disposé sur au moins une portion de la longueur de la surface intérieure d'au moins une paroi latérale du corps (2), dans le plan entre le cliquet (5) et la fente longitudinale (4) et est configuré pour engager de manière réversible l'aiguille dans la position intermédiaire, entre la position de sécurité et la position ouverte.

2. Gaine de sécurité selon la revendication 1, **caractérisée en ce que** le verrou longitudinal (6) est disposé sur la surface intérieure des deux parois latérales opposantes du corps (2).

3. Gaine de sécurité selon l'une quelconque des revendications 1-2, **caractérisée en ce que** le verrou longitudinal (6) s'étend le long de la longueur entière de la surface intérieure de la paroi latérale du corps (2).

4. Gaine de sécurité selon l'une quelconque des revendications 1-3, **caractérisée en ce que** le corps (2) présente une section transversale en forme d'un rectangle ou un trapézoïde.

5. Gaine de sécurité selon l'une quelconque des revendications 1-4, **caractérisée en ce que** l'élément de fixation d'aiguille (1) inclut un manchon d'assemblage (7) attaché au élément de fixation (1) sur sa extrémité première.

6. Gaine de sécurité selon la revendication 5, **caractérisée en ce que** des cannelures longitudinales (8) sont disposées sur la surface intérieure du manchon (7).

7. Gaine de sécurité selon l'une quelconque des revendications 5-6, **caractérisée en ce que** un bouchon (9) est inséré dans l'autre extrémité du manchon (7).

8. Gaine de sécurité selon l'une quelconque des revendications 1-7, **caractérisée en ce que** la paroi frontale (10) du corps (2) est tronquée vers la paroi supérieure du corps (2).

9. Gaine de sécurité selon l'une quelconque des revendications 1-8, **caractérisée en ce que** la charnière (3) inclut un élément résilient (11).

10. Gaine de sécurité selon l'une quelconque des revendications 1-9, **caractérisée en ce que** une saillie (12) est disposée sur la paroi supérieure du corps (2).

11. Gaine de sécurité selon la revendication 10, **caractérisée en ce que** des extrusions allongées (13) sont disposées sur les surfaces latérales extérieures des parois du corps (2) et/ou sur la paroi frontale (10) du corps (2) et/ou sur la saillie (12).

12. Gaine de sécurité selon la revendication 10, **caractérisée en ce que** c'est un composant intégral en matière plastique.

13. Ensemble d'aiguille avec une gaine de sécurité avec une gaine de sécurité pour fixer au connecteur de seringue, comprenant une aiguille à injection comprenant un bouchon (9) et un manchon (7) et la gaine de sécurité **caractérisé en ce que** la gaine de sécurité est une gaine de sécurité selon l'une quelconque des revendications 1-12.

14. Ensemble d'aiguille avec la gaine de sécurité selon la revendication 13, **caractérisé en ce que** le manchon (7) et le bouchon (9) sont des composants intégraux ou sont des éléments distincts fixés l'un dans l'autre.

15. Ensemble d'aiguille avec la gaine de sécurité selon l'une quelconque des revendications 13-14, **caractérisé en ce que** le bouchon (9) est un connecteur d'aiguille à injection.
